# EUROPEAN PATENT APPLICATION

(11) **EP 2 543 426 A1**
(43) Date of publication of application: **09.01.2013**
(21) Application number: 11710344.0
(22) Date of filing: 01.02.2011
(51) Int. Cl.: B01D 53/18, B01D 50/00, B01D 45/16, B01D 47/08

(54) **INDUSTRIAL GAS SCRUBBER**

(30) Priority: 01.02.2010 PT 10495910
(71) Applicant: Clearwinds-Systems S.A., 8600-069 Bensafrin (PT)
(72) Inventor: GOMES PEREIRA, Manuel, P-8550-454 Monchique (PT)
(74) Representative: Pereira da Cruz, Joao
(86) International application number: PCT/PT2011/000002
(87) International publication number: WO 2011/093735

(57) **Abstract**

The present invention relates to a domestic or industrial gas scrubber (16) to remove and separate impurities and fluids in a wet process, in the form of droplets and particles from gaseous streams of combustion gas, biogas or air, using a continuous colloidal centrifugal separating process. The droplets and particles are drawn by gravity to a settling tank (8), which includes a fan (11) ensuring the stream circulation inside the scrubber (16), and comprising two modules: one pulverization and absorption module (2), and another module (6) for moisture control and colloidal droplets capture, which were previously formed in module (2) in order to remove unwanted particles, this second module (6) being formed by sets of baffles (5) whose configuration forces the fluid stream to travel part of his path under a centrifugal effect.

## Description

### FIELD OF THE INVENTION

In recent years, the U.S. Department of Energy - DOE, the U.S. Environmental Protection Agency - EPA and also the European Environmental Protection Agency, have supported research to measure and control the emission of harmful pollutants, namely polycyclic aromatic hydrocarbons - PAH's from incomplete combustion of organic matter, such as, among others, coal-fired industrial boilers, wood pyrolysis for charcoal production, petroleum refining, domestic and industrial waste incineration, burning of organic matter from forests and fields, power generation via fossil fuel combustion, kerosene pyrolysis for the formation of benzene, toluene and other organic solvents, motor vehicles emissions, particularly diesel fueled.

Most of the heavy metals escape in the vapor phase and do not condense on ash particles at high temperatures, so this is the ideal opportunity to separate and remove them using the industrial gas scrubber described in the present invention.

The present invention relates, in general, with the cleaning methods domain for combustion gases, biogas, or air; and in particular with a method for removing impurities through the separation of the combustion gas produced during the consumption of fossil fuels, or the separation of solid waste from the digesters (Carbon and H₂S), or in air recirculation for heating, ventilation and air conditioning - HVAC systems, using two modules: one rotating pulverization module for absorption by saturation, and a second module consisting on sets of baffles, whose configuration forces the mass fluid (sludge) stream to travel part of its path under a centrifugal effect.

### TECHNICAL BACKGROUND

Prior art includes what is disclosed by patent applications Nos. W09951324, US4154191, BE806849, US2226127 and DE2548660.

W09951324 discloses a device for the removal by moist purification, combining the action of centrifugal effects which drags particles and other pollutants from a contaminated gas or vapor.

US4154191 discloses a gas flow purification system, particularly for the removal of Liquid and solid particles dragged into a gas flow.

BE806849 discloses a device for purifying pollutant gases, such as industrial boiler combustion gases, exhaust gas from internal combustion engines, etc., making them pass through a wet scrubber, followed by a cyclone separator.

US2226127 discloses a device for cleaning or purifying industrial gas or air, specifically a device which removes dust particles, first the thicker and then the thinner ones, fumes, gas, vapors and such, and finally tiny droplets which could be dragged by the gas.

DE2548660 discloses a device that eliminates the wet dust mist, particularly for mining extraction systems, using a contact area with the mist in which the drops of a liquid present in the gas flow can be separated using centrifugal forces.

According to the applicant's knowledge, there is no gas scrubber device or apparatus system, in the prior art, with the technical features described in the claims.

### SUMMARY OF THE INVENTION

The aim of the present invention is the removal of unwanted impurities from any combustion gas or air, preventing them to flow into the atmosphere.

One of the to modules that make the industrial gas scrubber, namely the module for moisture control and colloidal droplets capture, comprises a set of baffles with unique design, which highlights this invention as compared to conventional technologies.

The present invention also allows the monitoring of relative humidity percentage (RH) in the wet gas scrubber to the point of a dry flow at the outlet of the scrubber, reducing significantly the visible industrial plumes. Another distinguishing feature in this invention summary is that the module for moisture control and colloidal droplets capture has no moving parts or elements.

### BRIEF DESCRIPTION OF THE DRAWINGS

The description presented below makes reference to the attached drawings, which are presented only as a non-limiting reference, where:
- Figure 1 shows the domestic or industrial gas scrubber, and
- Figure 2 shows a top view of the module for moisture control and colloidal droplets capture, formed by baffles

### LEGEND OF THE FIGURES

- Polluted emissions inlet (1)
- Pulverization and absorption module (2)
- Recirculation pump (3) for fluid atomization
- Rotating atomization nozzle (4)
- Baffle (5)
- Module (6) for moisture control and colloidal droplets capture
- Filter-press (7) for contaminants recovery
- Settlement vessel (8) for collected impurities
- Ultra-violet (UV) lamp (9) for fluid sterilization
- Filter (10) consisting of organic product granules
- Fan (11)
- Outlet (12) for reutilizing 75% of the dry flow to destruct VOC's
- Outlet (13) for the release of 25% of exhausting gas free of fluid and molecular particles to the atmosphere
- Heat or cold pump (14)
- Sight hole (15)
- Scrubber (16).

### DETAILED DESCRIPTION OF THE INVENTION

One of the new features of the present invention is that the domestic or industrial gas scrubber (16) prevents the emission of polluted gas into the environment, thus providing cleaner air

This scrubber (16) is small in size when compared with other similar devices.

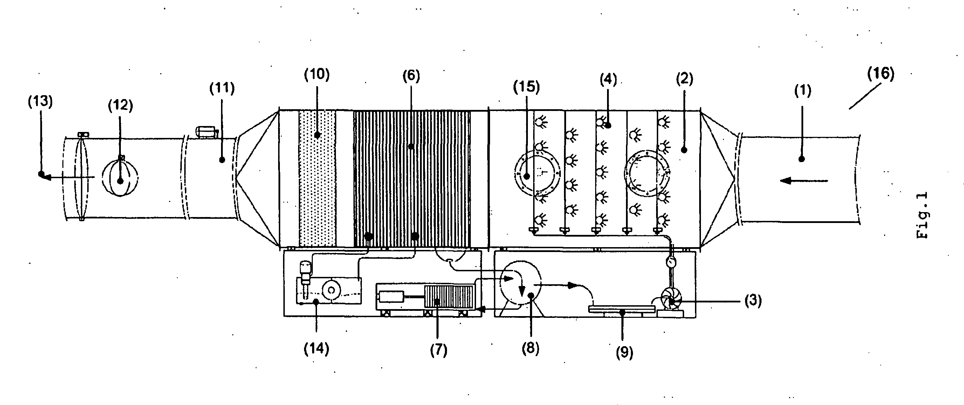

The scrubber (16) depicted in Figure 1 can be used for flows treatment, whether they come from combustion gas, gaseous emissions, biogas or air.

This scrubber (16) is also designed for heating or cooling air flows, previously cleaned, which can be reintroduced into the burner air intake of fossil fuels or biomass through the clean gas outlet (12).

This way the scrubber (16) will reuse more than 75% of the previously cleaned gas having his humidity controlled by module (6), allowing their reintroduction into the burner air intake of fossil fuels or biomass, through the clean gas outlet (12), for destruction of 75% of the volatile organic compounds (VOC's).

The remaining 25% of the previously cleaned gas having his humidity controlled by module (6) will leave the scrubber through outlet (13).

As shown in Figure 1, the scrubber (16) is designed to remove and separate impurities existing in gaseous streams of combustion gas, biogas or air in a wet process, using a colloidal separating process.

The scrubber (16) comprises a fan (11) that ensures fluid circulation inside it, and two modules, one pulverization and absorption module (2) and another module (6) for moisture control and capture of colloidal droplets formed in the pulverization module for the removal of unwanted particles. This second module (6) is formed by sets of baffles (5) whose configuration forces the fluid stream and sludge to travel part of his path under a centrifugal effect.

According to one embodiment of the present invention, the fan (11) can act as a suction fan or a blower, i.e. it can be installed at the inlet or at the outlet of the scrubber (16), depending on the percentage of fluid mass to be cleaned.

In the pulverization and absorption module (2) shown in Figure 1, the gas flow is humidified using rotating atomization nozzles (4), which create a continuous saturation and turbulent dispersion, so that the fluid with rotating atomization will absorb the particles to be subsequently removed at the module (6).

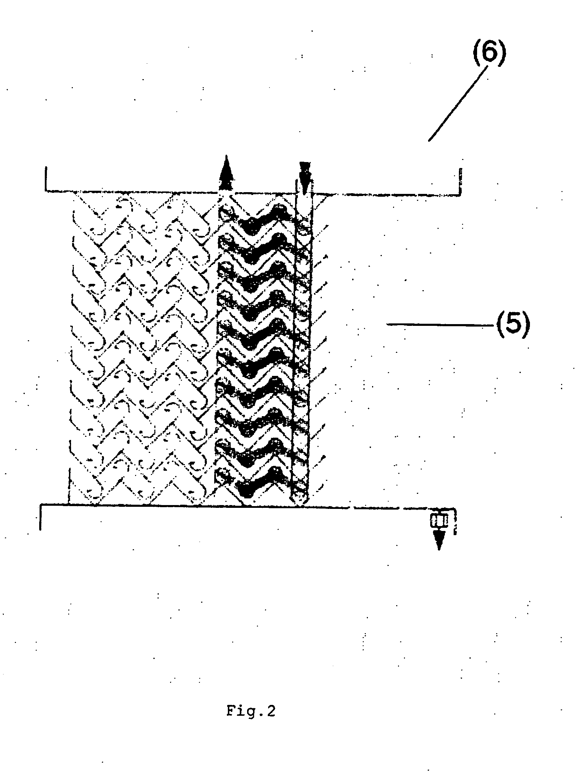

Figure 2 shows the module (6) formed by a set of baffles (5) to capture droplets and sludge consisting in one or more saturator stages, assuring that the air or gas stream at the scrubber outlets (13 and 12) has the desired humidity and is free from liquids or particles.

The saturators of the baffles (5) are unique design as ellipse arcs with a droplet stopper in order to produce the colloidal Centrifugal Effect and also control the humidity according to the needs, with zero moist carryover.

Depending on the type of gas to clean and on the desired quality for the cleaned air, the scrubber (16) may have more than one module (6) to capture droplets and sludge.

The scrubber (16) includes maintenance ports, not shown in the attached figures, for the removal of the module (6).

Another advantage of this scrubber (16) is that the fluid impurities and the sludge coming from the modules (2 and 6) are constantly eliminated.

In module (2), the sludge is deposited in the settlement vessel (8) for collected impurities, due to gravity and by the action of the fan (11) suction force.

In module (6), the sludge falls along the baffle (5) walls and into the settlement vessel (8) for collected impurities. Later, this sludge is pressed and filtered (7) for drainage and recovery of contaminants, which can be sterilized through an ultra-violet (UV) lamp (9) that will sterilize the fluids to be reused.

Fluids to be reused are introduced in module (2) using the recirculation pump (3) for fluid atomization.

The contaminants are removed in dry state and reused, for example in concrete mixture, etc.

The domestic or industrial gas scrubber (16) also comprises an absolute filter (10) , "Hepa filter", locate after the module (6), responsible for removing remaining particles smaller than one micron, including up to 0,012 microns.

The filter (10) contains organic product granules, a byproduct of the forestry industry, namely activated pine bark for heavy metal absorption, which acts as a final polishing in gaseous emissions or biogas flows.

The module (6) is formed by a set of fixed baffles (5), as shown in Figure 2, spaced between them depending on the type of emissions to clean, and can also include means for cooling and heating.

The heating or cooling is controlled by temperature control equipment (14) which increases or decreases liquid recirculation speed.

Said heating or cooling means are heat exchangers, consisting of tubes which are embedded into the baffles design.

These tubes are usually inserted at the core of the module (6) starting at the Baffle fourth stage of the ellipse arcs (5).

The quantity of heat exchangers varies, depending on dimensional requirements for each scrubber, (16).

When the scrubber is used for cleaning contaminated flue gas coming from any combustion process, the heating and cooling means, consisting on tubes existing in the baffles (5), will be used for re-circulating a cold fluid through the heat recuperator, using the high temperature from the emission source after cleaning.

In the module (2) of the domestic or industrial gas scrubber (16), depending on the pollutants to remove from the turbulent gas or other fluid, one or more chemical element is added, in order to promote adequate reactions to an easier absorption, capture and subsequent removal of unwanted materials.

The contaminated gases inlet (1) of the domestic or industrial gas scrubber (16) can be placed in vertical or horizontal position.

The same applies to clean gas outlet (13) of the domestic or industrial gas scrubber (16), which can also be placed in vertical or horizontal position.

Depending on the embodiment, the domestic or industrial gas scrubber (16) can be placed vertically or horizontally.

As will be clear to an expert, several changes in details are possible; however they must be included in the scope of this invention.

The invention should then be limited only by the spirit of the following claim.

## Claims

1. Domestic or industrial gas scrubber (16) to remove and separate impurities through a wet process, in the form of droplets from gaseous streams after being properly wetted and saturated, existing in gaseous streams of combustion gas, biogas or air, using a continuous colloidal separating process, and which includes a fan (11) ensuring the circulation inside it of liquid or gas fluids, air or gas flows, **characterized in that** it comprises two modules: one pulverization and absorption module (2), and another module (6) for moisture control and colloidal droplets capture, which were previously formed in the pulverization module (2) in order to remove unwanted particles, this second module (6) being formed by baffles (5) whose configuration forces the fluid flow to travel part of his path under a centrifugal colloid effect.

2. Domestic or industrial gas scrubber (16) according to claim 1, **characterized in that** the gaseous fluid is cleaned in the pulverization and absorption module (2) in a wet process using rotating atomization nozzles (4) for pulverization, creating a saturation and absorption phase of the pollutants.

3. Domestic or industrial gas scrubber (16) according to claim 1, **characterized in that,** in the module (6) for moisture control and colloidal droplets capture, each baffle (5) has a curved shape similar to a hook, and **in that** these baffles are grouped into sets consisting of stages to ensure a flow rate with the desired humidity at the outlet (13).

4. Domestic or industrial gas scrubber (16) according to claim 1, **characterized in that** it comprises heating and cooling means placed on the module (6) core, consisting of tubes embedded into the baffles.

5. Domestic or industrial gas scrubber (16) according to claim 4, **characterized in that** the cooling and heating means are heat exchangers.

6. Domestic or industrial gas scrubber (16) according to claim 4, **characterized in that** the quantity of heat exchangers varies, depending on dimensional requirements for each scrubber (16).

7. Domestic or industrial gas scrubber (16) according to claim 1, **characterized in that** in module (2), depending on the pollutants to absorb and saturate from the gas, air, or fluid flows, one or more chemical elements is added to promote adequate reactions for easier absorption, saturation and subsequent removal of the remaining unwanted particles at the module (6).

8. Domestic or industrial gas scrubber (16) according to claim 1, **characterized in that** it comprises means for the continuous evacuation of matter, sludge and fluids removed at the module (6), consisting of:
- a settlement vessel (8) for collected impurities, that collects fluid from filter-press (7), module (6) and module (2);
- a filter-press (7) for compacting contaminant matter and recovery of reagents and liquids;
- an ultra-violet (UV) lamp (9) for sterilization of atomization fluids to be reintroduced in module (2), and
- a recirculation pump (3) to reintroduce in module (2) the atomization fluid coming from the vessel (8), after being sterilized by ultra-violet (UV) lamp (9).

9. Domestic or industrial gas scrubber (16) according to claim 1, **characterized in that** it comprises an absolute filter (10) located after the modules (6), responsible for removing unwanted particles smaller than one micron.

10. Domestic or industrial gas scrubber (16) according to claim 1, **characterized in that** his placement is easily achieved in a vertical or horizontal plan.

11. Domestic or industrial gas scrubber (16) according to claim 1, **characterized in that** it reuses more than 75% of the previously cleaned gas having his humidity controlled by module (6), allowing their reintroduction into the burner air intake of fossil fuels or biomass through the outlet (12), for destruction of 75% of the volatile organic compounds (VOC's), and **in that** the remaining 25% of the previously cleaned gas having his humidity controlled by module (6) leave the scrubber, through outlet (13).
